# EUROPEAN PATENT APPLICATION

(11) **EP 4 813 315 A1**
(43) Date of publication of application: **30.09.2026**
(21) Application number: 26168548.1
(22) Date of filing: 27.03.2026
(51) Int. Cl.: A61B 10/02

(54) **SINGLE ACTION FULL CORE BIOPSY DEVICE**

(30) Priority: 28.03.2025 US 202563779506 P; 25.03.2026 US 202619578621
(71) Applicant: RLS Interventional, Inc., Grand Rapids, Michigan 49512 (US)
(72) Inventor: FIELD, Steven E, Grand Rapids, 49512 (US); SCHAFTENAAR, John, Grand Rapids, 49512 (US); GRASHORN, Patrick, Grand Rapids, 49512 (US); DAY, Morgan, Grand Rapids, 49512 (US); FASE, Corey, Grand Rapids, 49512 (US)
(74) Representative: Forresters IP LLP

(57) **Abstract**

A biopsy device and method for taking a tissue sample with a biopsy device having a stylet, a spoon cannula, and a cutting cannula. The cutting cannula can be manually armed while manually arming the coring cannula. The manual advancing of the coring cannula can automatically advance the cutting cannula.

## Description

### FIELD

The present invention relates to a biopsy device.

### BACKGROUND

It is frequently necessary to sample or remove a tissue sample from a suspect tissue for testing. In humans, such a sample removal is particularly useful in the diagnosis and treatment of cancerous or pre-cancerous conditions. In the case of suspected cancer, particularly breast cancer, early detection and diagnosis is helpful to the success of the patient's treatment and recovery.

Various techniques are available to aid in detection and diagnosis, including physical examination and imaging, such as mammography, x-ray, ultrasound, magnetic resonance imaging (MRI), and the like. When a condition is detected that suggests the possibility of cancer, a biopsy can be performed to obtain tissue samples for a complete diagnosis.

One biopsy technique frequently performed is a core biopsy, which uses a core biopsy device in which a cannula is inserted into the tissue of interest, thereby coring a biopsy sample from the tissue having a cross section similar to that of the cannula, and which is retained within the cannula. The cannula, with the biopsy sample, is then removed from the tissue, followed by cytological and/or histological analysis of the sample.

One group of core biopsy devices is based on the combination of a notched inner stylet and an outer severing cannula. The stylet is retained within the lumen of the outer cannula such that the pointed end of the stylet closes off the open end of the cannula. The stylet and cannula are advanced into the tissue mass until they are near the desired biopsy site. The stylet is then advanced relative to the outer cannula to expose the notch to the biopsy site where the tissue prolapses into the notch. The outer cannula is then advanced to sever the tissue in the notch. The disadvantage of this method is that it produces a small core biopsy, referred to as a "partial core" biopsy, relative to the outer cannula size since the cross section of the sample is substantially equal to the cross section of the stylet notch, which is substantially smaller than the cross section of the outer cannula. The advantage of this method is that the sample is completely severed from the tissue mass and securely retained within the notch.

Another group of core biopsy devices is based on a coring cannula in combination with a non-notched stylet. The stylet is used to close the end of the coring cannula during the insertion of the coring cannula into the tissue adjacent the biopsy site. The coring cannula is then advanced relative to the stylet into the biopsy site to retain a sample within the coring cannula. The advantage of this device is that a full core biopsy sample is obtained. That is, the "full core" sample has a cross section that is substantially equal to the cross section of the coring cannula. The full core sample provides a much larger sample which is highly advantageous.

The disadvantage of the full core device is that the end of the sample is not positively severed from the tissue mass, creating the possibility that the biopsy sample will be pulled out of the coring cannula upon the withdrawal of the coring cannula. This can happen if the forces holding the sample in the coring cannula are not sufficient to tear the end of the sample from the tissue mass. Since the sample normally comprises wetted tissue that completely fills the coring cannula, the suction force and/or the frictional force between the tissue sample and the inner wall of the coring cannula are the dominant forces for retaining the sample in the cannula. However, if these forces are not sufficient to tear the end of the sample from the tissue mass, the sample will be pulled out of the coring cannula upon the removal of the coring cannula. Some practitioners pivot the biopsy device in hopes that the sharpened end of the cannula will at least partially sever the attached portion of the sample, making it more likely the tissue sample will be removed. However, this is not preferred as it increases the damage to the remaining tissue.

Attempts have been made to improve the severing of the sample from the tissue mass for the full core device. In some cases, the interior of the coring cannula is provided with a raceway in which a severing finger could be advanced/retracted. After the advancing of the cannula to take the core sample, the severing finger is advanced, guided by the raceway, to sever the tissue. In some cases, when the finger is advanced, it closes the end of the coring cannula and is left in the advanced position during removal. A disadvantage of this method is that the sample is not truly a full core sample since part of the interior of the cannula was reserved for the raceway. If the sample produced by the cannula with the raceway was the same size as the full core sample, the cannula with the raceway would require a larger cross-sectional cannula, which is not desirable. In the biopsy art, it is highly desirable to minimize the cross section of the cannula to minimize the damage to the surrounding tissue and to minimize the invasiveness of the procedure. Generally, the smaller the cross section, the less pain the patient experiences after the procedure, and the more desirable the device.

Another alternative to severing the sample end in a full core device comprises the addition of a cutting cannula that circumscribes the coring cannula. The cutting cannula has a cutting element that severs the sample within the coring cannula or at the tip of the coring cannula. The cutting cannula can include a finger that extends over the end of the coring cannula or passes through a window near the end of the coring cannula.

### BRIEF SUMMARY

This description generally relates to a device and method of taking a tissue sample with a needle assembly having a stylet, a coring cannula, and a cutting cannula, by manually arming the cutting cannula; manually advancing the coring cannula; and automatically advancing the cutting cannula by the manual advancement of the coring cannula. The manual advancement of the coring cannula and the automatic firing of the cutting cannula can be done in a single action of the device. The single action can include the manual movement of a plunger coupled to the coring cannula.

### BRIEF DESCRIPTION OF THE DRAWINGS

In the drawings:
Fig. 1 is a perspective view of a full core biopsy device according to the present disclosure with an actuator assembly and a needle assembly having a stylet, coring cannula, and cutting cannula.
Fig. 2 is an exploded view of the full core biopsy device from FIG. 1 illustrating the needle assembly and the components of the actuator assembly: upper housing, lower housing cutting cannula hub, coring cannula hub, stylet hub, spring, and plunger.
Fig. 3 is a bottom perspective view of the cutting cannula hub.
Fig. 4 is a top view of the actuator assembly with the upper housing removed to see the relative locations of the cutting cannula hub, coring cannula hub, stylet hub, spring, and plunger during a resting state.
Figs. 5-9 illustrate some of the operational positions of the biopsy device including an enlarged view of the tip of the needle assembly corresponding to that operational position.

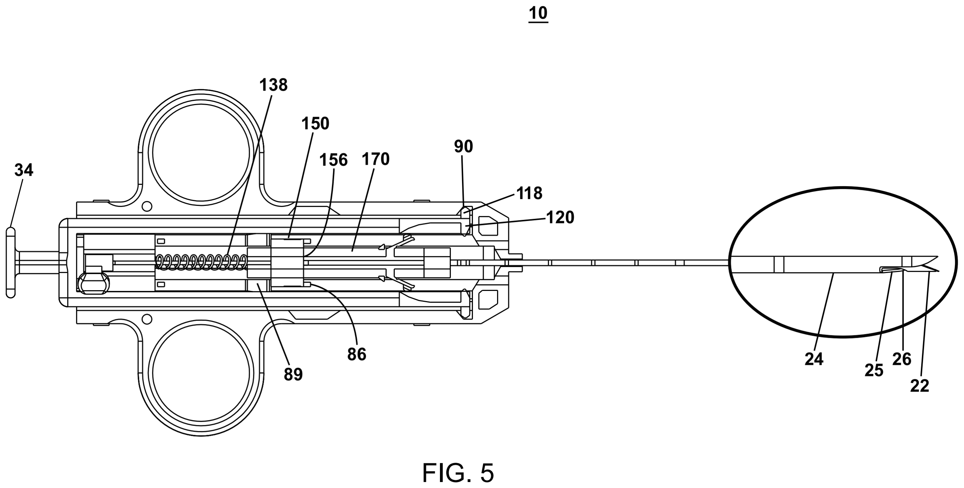

Fig. 5 is a longitudinal sectional view of the biopsy device in a resting state.

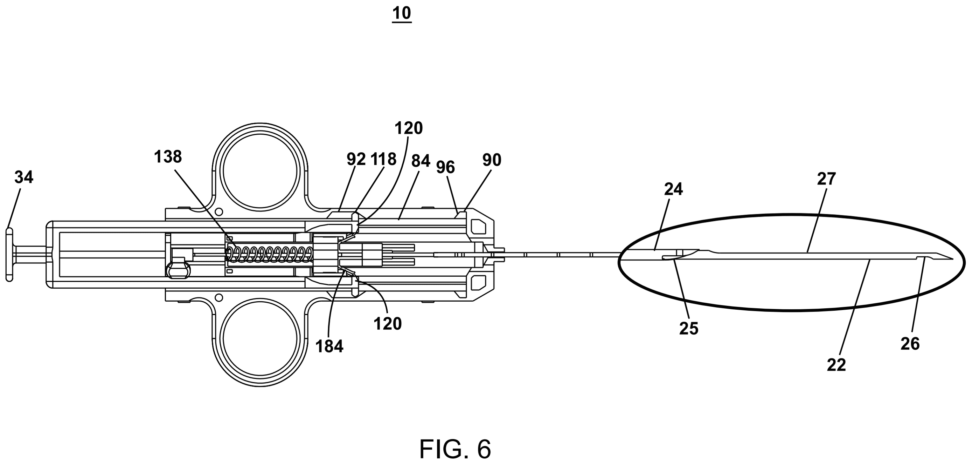

Fig. 6 is a longitudinal sectional view of the biopsy device when the cutting cannula is in an armed state.

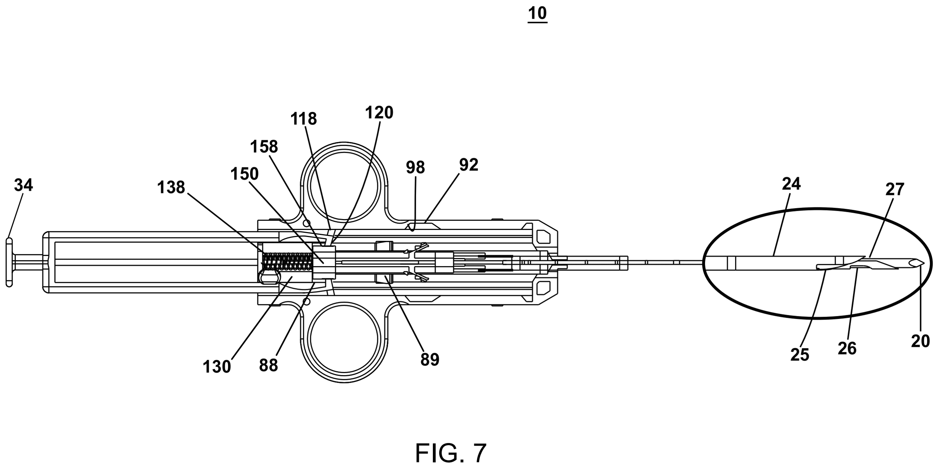

Fig. 7 is a longitudinal sectional view of the biopsy device when the coring cannula is in an armed state.

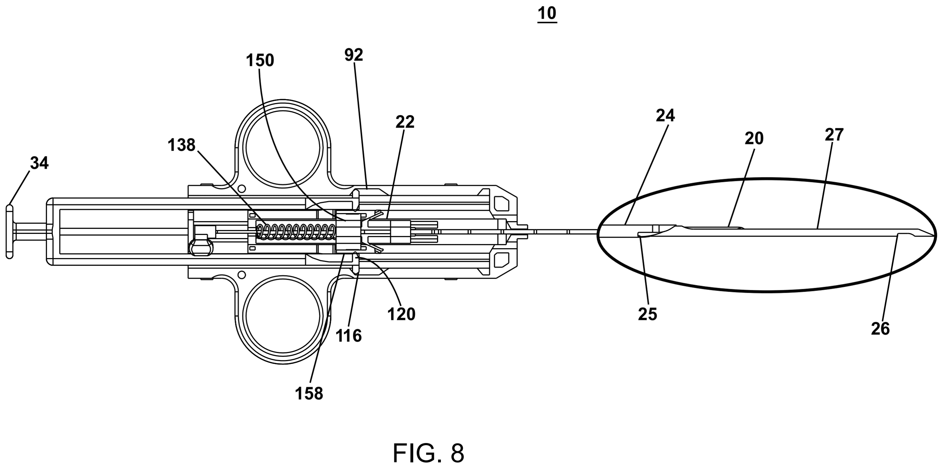

Fig. 8 is a longitudinal sectional view of the biopsy device when the coring cannula is in a cored state where the coring cannula has been advanced and the core is formed, but not yet severed.

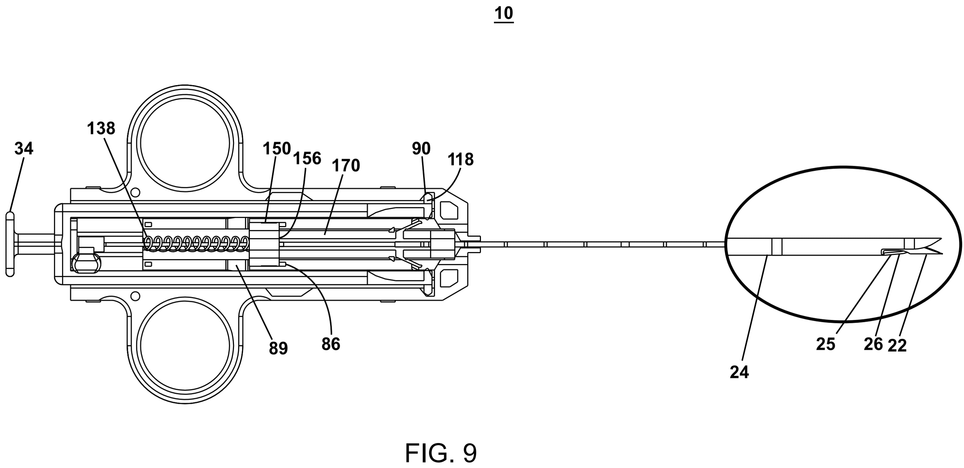

Fig. 9 is a longitudinal sectional view of the biopsy device when the cutting cannula is in a cut state and has severed the core.

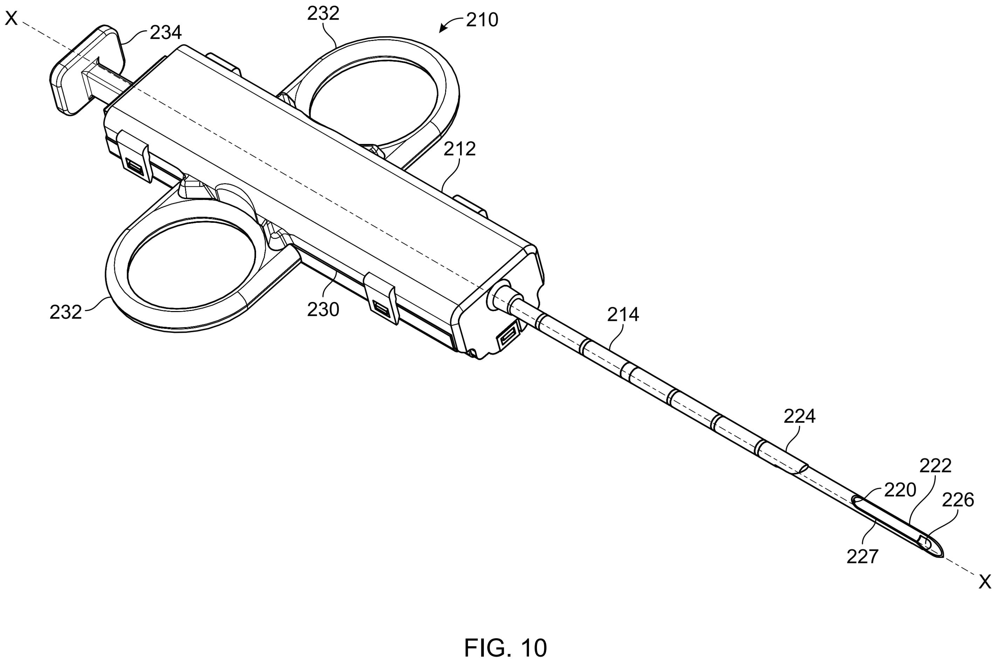

Fig. 10 is a perspective view of a full core biopsy device according to a second embodiment of the present disclosure with an actuator assembly and a needle assembly having a stylet, coring cannula, and cutting cannula.

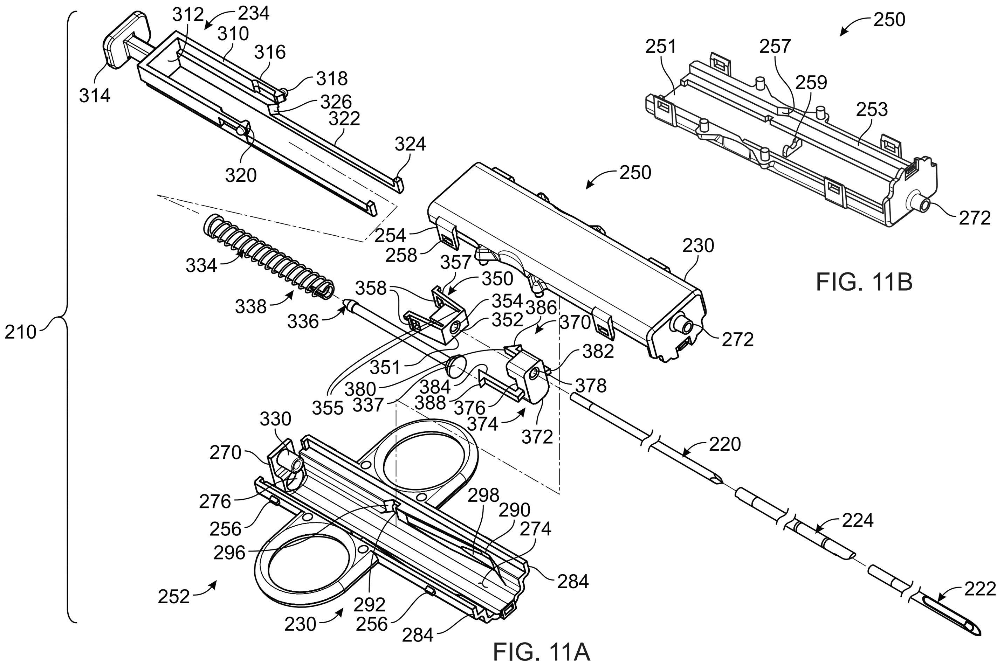

Figs. 11A-C are exploded views of the second embodiment of the full core biopsy device from FIG. 10 illustrating the needle assembly and the components of the actuator assembly: upper housing, lower housing cutting cannula hub, coring cannula hub, stylet hub, spring, and plunger.

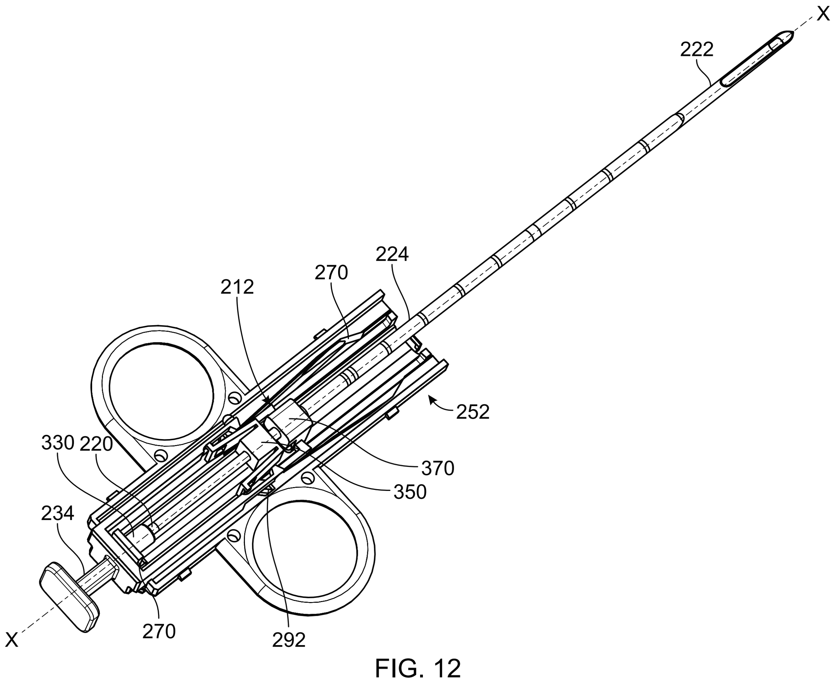

Fig. 12 is a top view of the actuator assembly of the second embodiment with the upper housing removed to see the relative locations of the cutting cannula hub, coring cannula hub, stylet hub, spring, and plunger during an armed state.

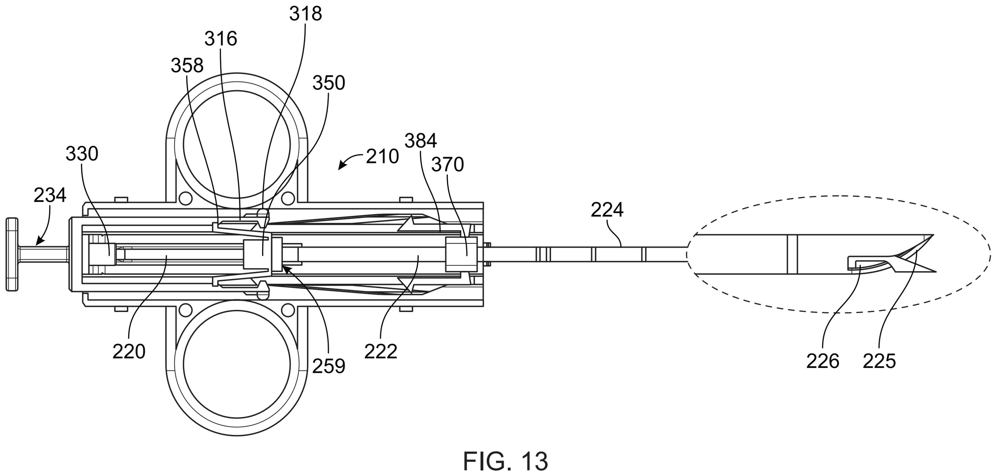

Figs. 13-19 will follow some of the more notable operational positions of the biopsy device including an enlarged view of the tip of the needle assembly corresponding to that operational position.
Fig. 13 is a longitudinal sectional view of the second embodiment biopsy device in a resting state.

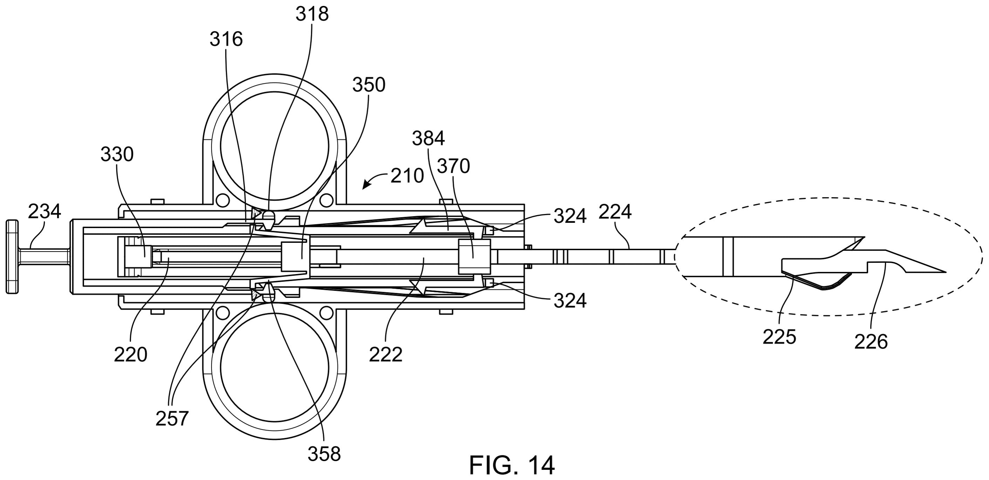

Fig. 14 is a longitudinal sectional view of the second embodiment biopsy device when the cutting cannula is starting an arming cycle.

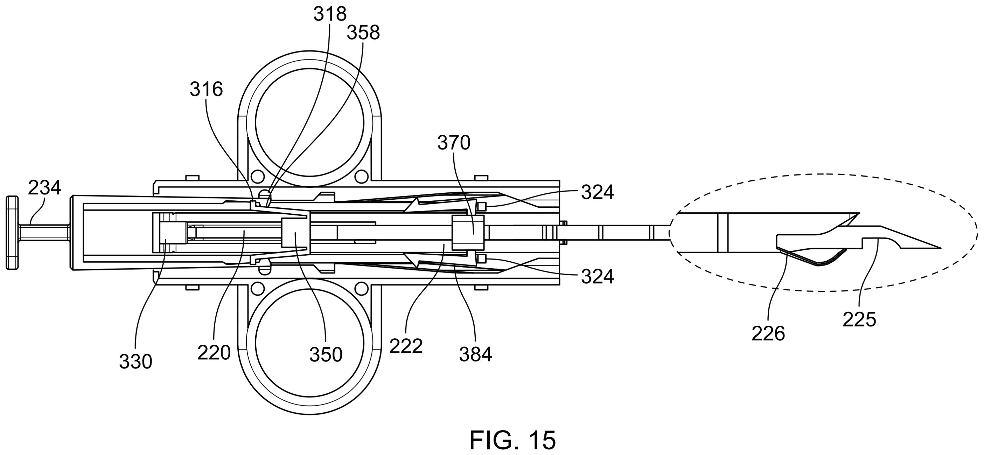

Fig. 15 is a longitudinal sectional view of the second embodiment biopsy device when the cutting cannula is in a dwell as arming continues.

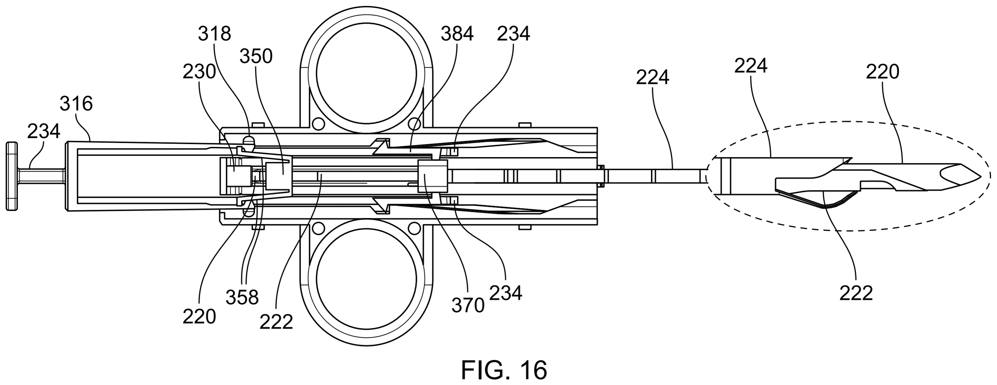

Fig. 16 is a longitudinal sectional view of the second embodiment biopsy device when the coring cannula and the cutting cannula are in a fully armed state.

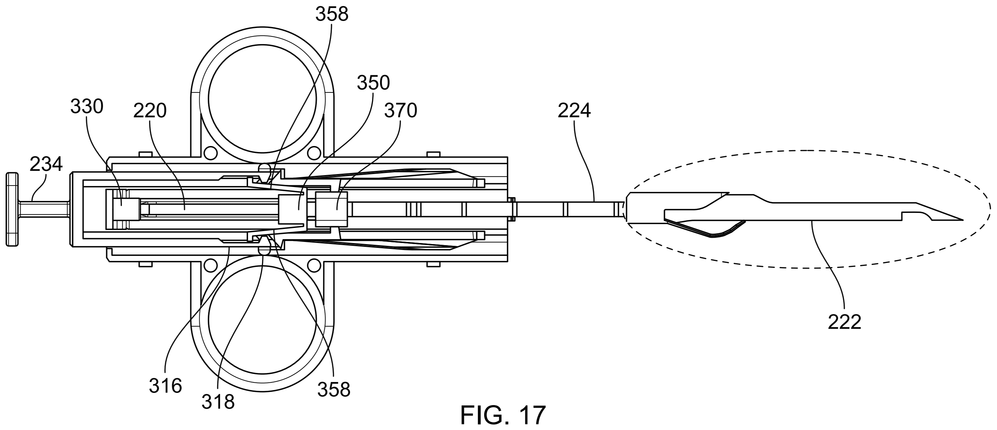

Fig. 17 is a longitudinal sectional view of the second embodiment biopsy device when the plunger is advanced forward.

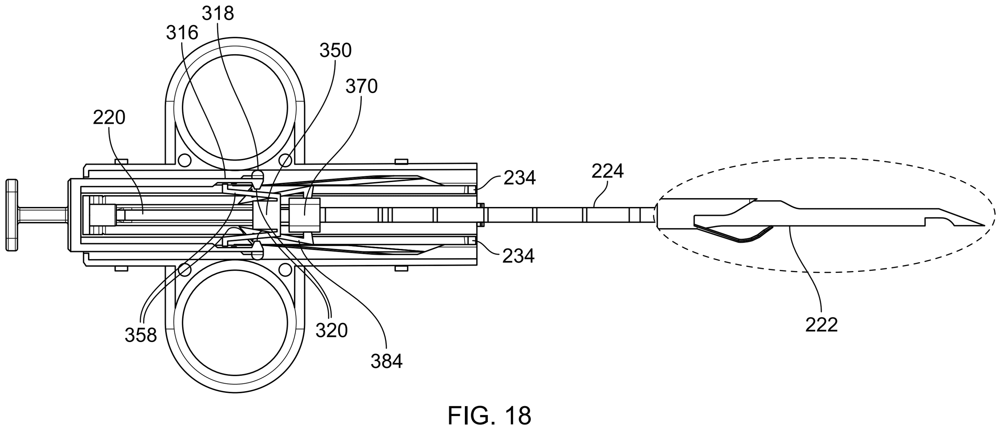

Fig. 18 is a longitudinal sectional view of the second embodiment biopsy device when the plunger reaches resistance location.

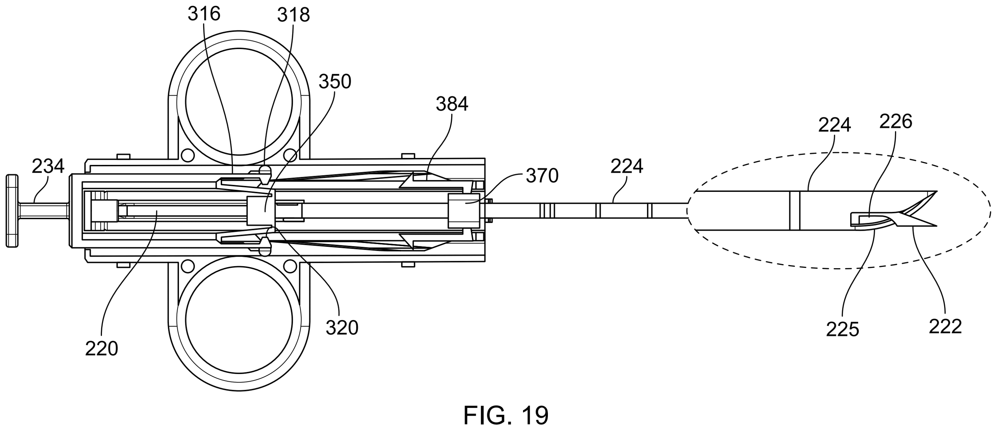

Fig. 19 is a longitudinal sectional view of the second embodiment biopsy device when the plunger moves past resistance location and the cutting cannula is in a cut state and has severed the core.

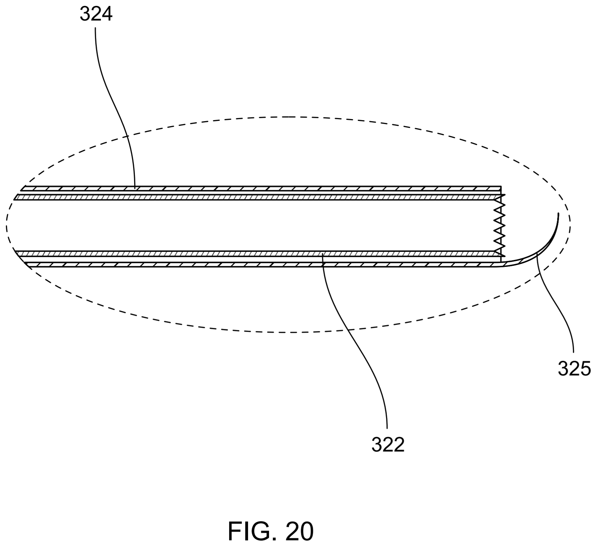

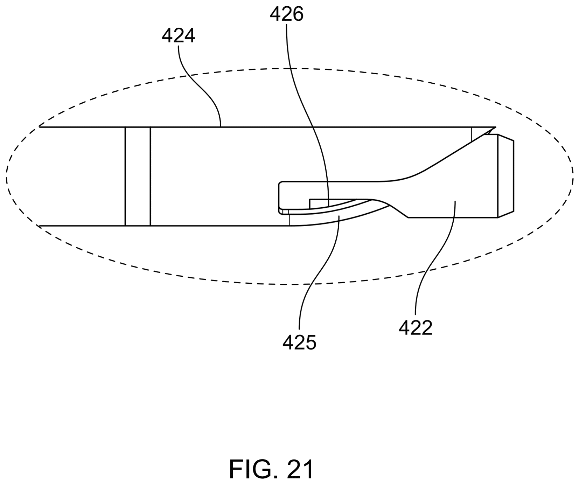

Figs. 20 and 21 illustrate views of alternative biopsy needle tip assemblies.

The figures referred to above are not necessarily drawn to scale, should be understood to provide a representation of particular embodiments of the disclosure, and are merely conceptual in nature and illustrative of the principles involved. Some features depicted in the drawings may have been enlarged or distorted relative to others to facilitate explanation and understanding.

### DETAILED DESCRIPTION

The invention relates to biopsy devices for obtaining biopsy samples from tissue. In one of its aspects, the invention relates to a full core biopsy device for obtaining biopsy samples from tissue, and more particularly, to a full core biopsy device having a needle assembly comprising a stylet, coring cannula, and a cutting cannula. In taking a specimen with this needle assembly, the coring cannula is advanced beyond the stylet to form a core of tissue within the coring cannula. The cutting cannula is then advanced relative to the cutting cannula to sever the core of tissue from the tissue mass. Previous biopsy devices using this type of needle assembly have relied on an automatic firing of the biopsy device, where upon the actuation of the biopsy device by a user, the coring cannula is automatically advanced, such as by spring force, beyond the stylet, followed by a fully automatic advancing of the cutting cannula to sever the tissue mass. The prior biopsy devices are typically manually charged, such as with a cocking device, which moves the coring cannula and cutting cannula to their charged positions and compresses the spring or springs used for actuation.

Some practitioners do not prefer these fully automatic biopsy devices because they do not provide any tactile response as the coring cannula is being advanced. Thus, the user cannot "feel" how easy or difficult it is to advance the coring cannula, which may give the user insight, beyond what is available by an imaging device such as ultrasound, into the nature of the tissue mass or the position of needle assembly within the tissue mass. The fully automatic biopsy devices also do not let the user adjust the position of the device as the coring cannula is advanced.

A manual advancement of the coring cannula can provide the desired tactile feel and readjustment of the biopsy device as the coring cannula is advanced. A device with manual advancement should take into account the convenience of the user, especially convenience that will lead to accurate positioning of the biopsy device. As the user only has two hands to operate the device, care must be taken to ensure that the manual charging of the device and the manual advancement of the coring cannula can be done in a manner that lets the user maintain a hold of the biopsy device with one hand while charging and advancing with the other hand. The disclosed biopsy device provides the user with these benefits, along with providing a charging and advancing that is longitudinally aligned with the direction of travel axis for the coring cannula and cutting cannula, making it much less likely that the charging and advancing will cause an inadvertent change in position of the biopsy device.

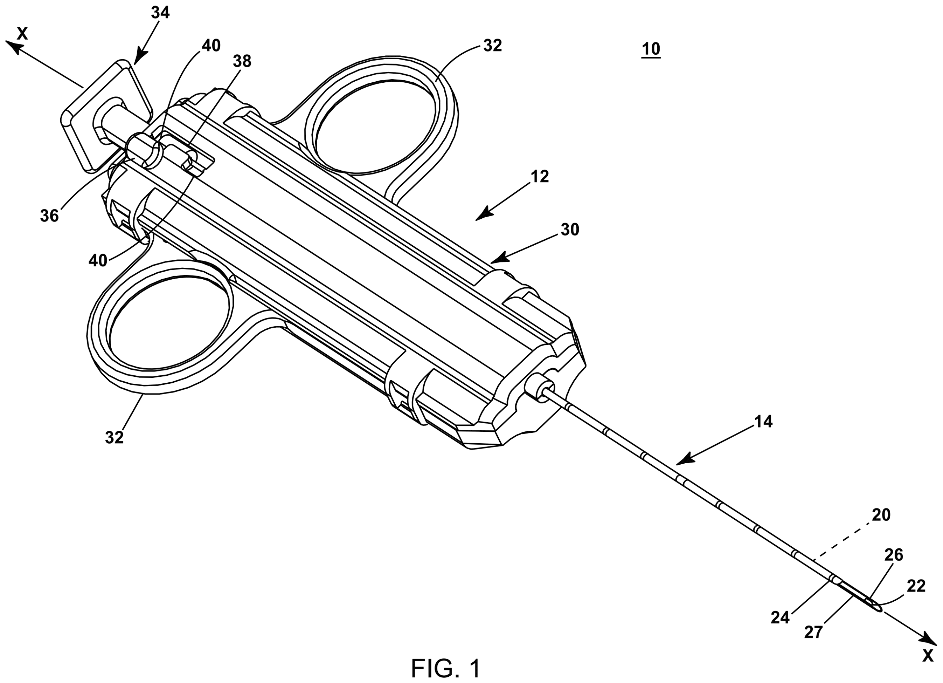

FIG. 1 is a perspective view of a full core biopsy device 10 according to the present disclosure. The full core biopsy device 10 can include an actuator assembly 12 structurally and operably connected to a needle assembly 14. The needle assembly 14 can be utilized to penetrate a tissue mass for obtaining a core biopsy sample, alternately referred to as a biopsy specimen, from a target area in the tissue mass, such as a lesion. The needle assembly includes a stylet 20, coring cannula 22, and cutting cannula 24. The coring cannula 22 can have a window 26 and the cutting cannula 24 can have a cutting finger 25 that passes through the window 26 to sever a tissue sample in the coring cannula 22. The coring cannula 22 can further have a spoon 27. One or both of the coring cannula 22 or the cutting cannula 24 can have a sharpened tip. A suitable needle assembly is disclosed in U.S. Patent No. 11,510,661, and specifically to the needle assembly and the manner of movement of the needle assembly to take a tissue specimen.

An embodiment of the actuator assembly 12 is described and illustrated herein comprising a manually operated, single action hand-held device capable of controlling the acquisition and removal of a tissue specimen, which can be a core biopsy sample from the lesion. By single action, it is meant that the manual advancement of the coring cannula will trigger the automatic firing of the cutting cannula. Thus, one single manual action accomplishes both the coring and the cutting of the tissue specimen.

The biopsy device 10 has an operational axis X defined by the needle assembly 14 and extending through the actuator assembly 12. The axis X also defines an insertion axis of the needle assembly 14 into the tissue mass. The axis X need not be centered on the actuator assembly 12. As shown, the axis X is off center of the geometric center of the end of the actuator assembly 12. The actuator assembly 12 comprises a body 30, which can function as a grip or handle, from which a pair of finger grips 32 extend. A manual actuator, in the form of a plunger 34 extends from the body 30 opposite the needle assembly 14. An optional specimen length selector 36 projects through a slot 38 in the body 30, with the slot defining two detents 40.

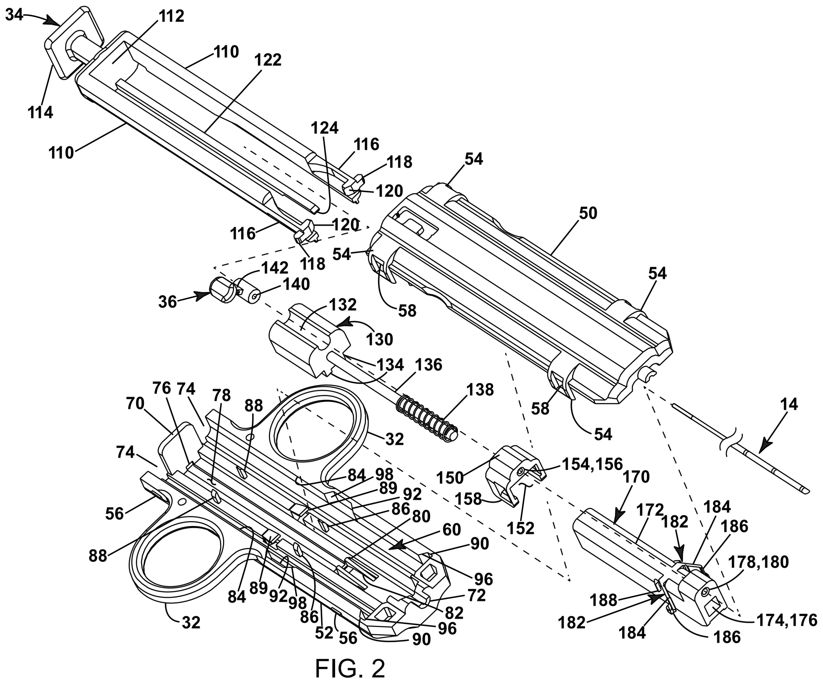

Referring to Fig. 2, the major elements of the actuator assembly 12 are better seen and understood. The body 30 is formed by a housing having an upper housing 50 and a lower housing 52. A series of spring clips in the form of a finger 54 and tab 56 are provided to secure together the upper housing 50 and the lower housing 52. The finger 54 has a slot 58 that receives the tab 56. The finger 54 is resilient, permitting it to deflect over the tab 56 to position the tab within the slot 58 when the upper and lower housings 50, 52 are snapped together as seen in Fig. 1. When snapped together, the upper and lower housings 50, 52 define an interior 60.

The lower housing 52 has an end plate 70, at a rear end, and a needle guide 72, at the front end or nose of the body. Arm channels 74 are located on opposite sides of the end plate 70. A key slot 76 is provided in the end plate 70 and connects to a keyway 78, which extends up to a cutting cannula catch in the form of a deflectable ramp 80. The deflectable ramp 80 is a split ramp that defines a key channel 82 forming an extension of the keyway 78. A pair of sidewalls 84 are located on opposite sides of the keyway 78 and have forward and rearward stops 86, 88, along with deflectable stops 89 projecting from the sidewalls 84. First and second relief channels 90, 92 are formed in the sidewalls 84 of the lower housing 52 and are coupled to the sidewalls 84 by first and second plunger deflectors 96, 98.

The plunger 34 comprises a pair of arms 110 connected by a plunger hub 112 from which a nob 114 extends. The plunger arms 110 terminate in fingers 116 having an outwardly directed follower 118 and an inwardly directed coring hub strike 120. A key 122 extends from the plunger hub 112 and is vertically below the plunger arms 110 and terminates in a key tip 124.

A stylet hub 130 has a length adjustment channel 132 on an upper side and a pair of guide rails 134 on a lower side. A spring guide 136, about which a coil spring 138 is wound, projects forward from a front face of the stylet hub 130. The specimen length selector 36 includes a stylet seat 140 in which a rear end of the stylet 20 is mounted. A selector lever 142 extends from the stylet seat 140.

A coring cannula hub 150 has an inverted U-shaped body defining a coring cannula passage 152 and having a coring cannula seat 154 having a through passage 156, and a pair of plunger catches formed by catch openings 158 in the sides of the U-shaped body. The catch openings 158 can extend to the coring cannula channel 152. A rear end of the coring cannula 22 is mounted to the through passage 156, such as by press-fitting.

A cutting cannula hub 170 comprises a body 172 with a spring guide receiver 174 in the form of an internal channel 176, and a cutting cannula seat 178 with a through passage 180. A pair of resilient catches 182 extend from the side of the body 172. The resilient catches 182 include resilient wings 184 that terminate in tips 186. A set of wedges 188 extend from the body 172 at a location rearward of the resilient catches 182. The cutting cannula 24 can be mounted to the cutting cannula seat 178, such as, by press-fitting a rear end of the cutting cannula 24 into the cutting cannula through passage 180.

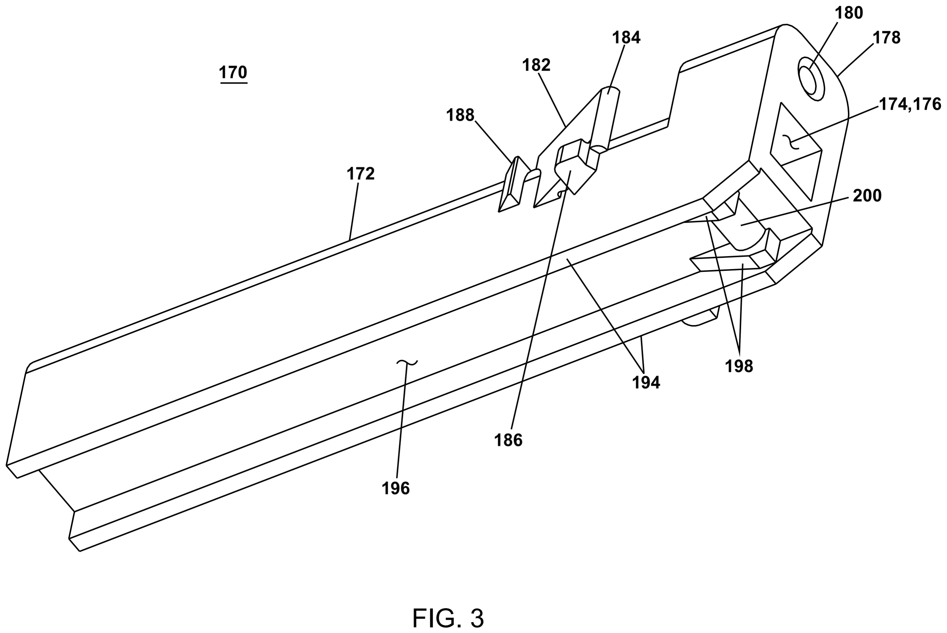

Referring to Fig. 3, the body 172 includes spaced guide rails 194 defining a channel 196, which is complementary to the keyway 78. At a forward end of the channel 196 lies a split deflector ramp 198 with an interstitial rib 200.

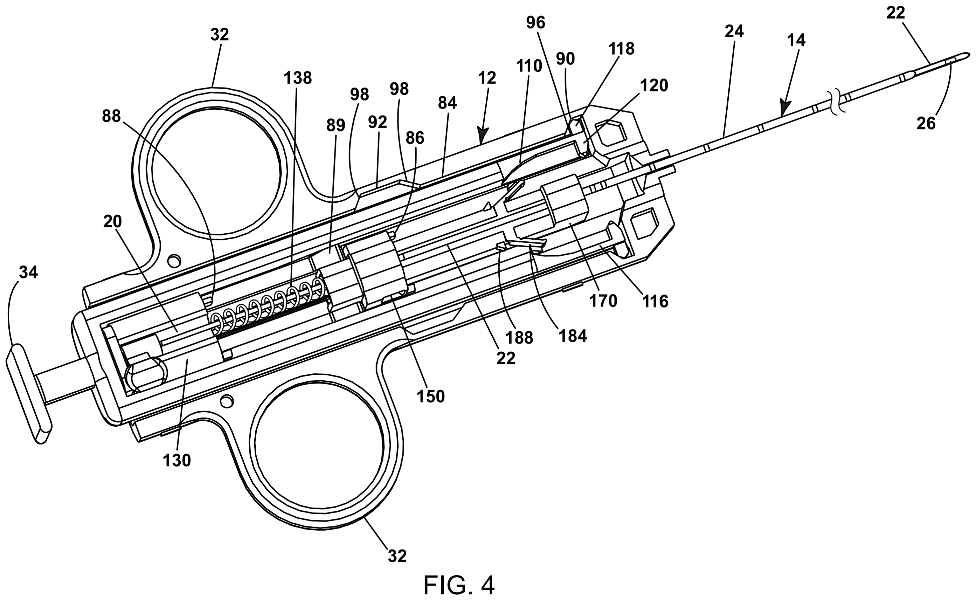

Referring to Fig. 4, the actuator assembly 12 is shown with the upper housing 50 removed to better see the locations of the plunger 34, stylet hub 130, coring cannula hub 150, and cutting cannula hub 170 when the actuator assembly 12 is in a rest state. As is seen, the plunger 34 is located in the lower housing 52 such that the plunger arms 110 pass through the arm channels 74 and the key 122 passes through the key slot 76 and will reside within the keyway 78.

The stylet hub 130, when positioned within the lower housing, resides between the end plate 70 and the rearward stops 88, with the guide rails 134 located between the sidewalls 84, with the space between the guide rails overlying the keyway 78. The spring guide 136 projects toward the nose of the body 30.

When the coring cannula hub 150 is positioned within the lower housing 52 it resides between the forward stops 86 and the deflectable stops 89. In this position, the through passage 156 is in alignment with the stylet seat 140 and the stylet 20 can pass through the through passage 156 and into the interior of the coring cannula 22.

When the cutting cannula hub 170 is positioned within the lower housing 52, it resides between the sidewalls 84 with the channel 196 overlying the keyway 78 and the deflectable ramp 80, which is received within the channel 196. The body of the cutting cannula hub 170 is aligned with and free to move through the coring cannula passage 152. The cutting cannula through passage 180 is aligned with the coring cannula through passage 156 thereby permitting the insertion of both the coring cannula 22 and the stylet 20 residing within the cutting cannula 24.

The operation of the biopsy device 10 will be described with reference to Figs. 5-9. For Figs. 5-9, only the part numbers relevant to the corresponding description are illustrated in the drawing to avoid overcrowding the drawing with part numbers to improve the view of the part. In Fig. 5, the biopsy device 10 is shown in a rest state, where the plunger 34 is fully inserted such that the followers 118 are received within the first relief channels 90. In this position the plunger fingers 116 are substantially undeflected, the coil spring 138 is abutting and biasing the cutting cannula hub 170 forward into abutment with the nose, the wings 184 reside axially behind the coring hub strikes 120, the body of cutting cannula hub 170 is aligned with and at least partially resides within coring cannula through passage 156, and the coring cannula hub 150 lies between the forward stops 86 and the deflectable stops 89. In this position, the coring cannula 22 and cutting cannula 24 are fully forward, with the cutting finger 25 of the cutting cannula 24 extending through the window 26 of the coring cannula 22. The tip of the stylet 20 is not visible and is within the coring cannula 22, spaced axially from the spoon 27.

Fig. 6 shows the biopsy device with the cutting cannula 24 in the armed state. To arm the cutting cannula from the rest position, the user will pull rearwardly on the plunger 34 causing the followers 118 to follow along the first deflectors 96 and move from the first relief channel 90 onto the sidewalls 84, which causes the plunger fingers 116 to deflect inwardly. The user continues to pull the plunger rearwardly until the followers 118 reach the second relief channel 90, where the resilient nature of the plunger fingers 116 biases the followers 118 into the second relief channel 92.

During this rearward movement of the plunger 34 several things take place. The inward deflection of the plunger fingers 116 causes the strikes 120 to catch the wings 184 thereby causing the cutting cannula hub 170 to travel rearwardly with the plunger 34. The rearward movement of the cutting cannula hub 170 compresses the coil spring 138. The rearward movement of the cutting cannula hub 170 also causes the split deflector ramp 198 on the bottom of the cutting cannula hub 170 to ride over and temporarily deflect downwardly the deflectable ramp 80, until the split deflector ramp 198 moves rearward beyond the deflectable ramp 80. Right as, or just before, the followers 118 reach the second relief channel 92, the split deflector ramp 198 is past the end of the deflectable ramp 80, which returns to its undeflected position in front of the split deflector ramp 198. The deflectable ramp 80 and split deflector ramp 198 form a latch for the cutting cannula hub 170 with the deflectable ramp 80 forming the catch and the split deflector ramp 198 forming the strike. This latch prevents the force of the now compressed coil spring from moving the cutting cannula hub 170 forward. Finally, as the followers 118 fully deflect into the second relief channel 92, the strikes 120 disengage from the wings 184, with only the latch formed from the deflectable ramp 80 and split deflector ramp 198 holding the cutting cannula hub against the biasing force from the coil spring 138, thereby placing the cutting cannula in the armed state.

It should be noted that the movement of the followers 118 into the second relief channels 92 and the latching of the split deflector ramp 198 and deflectable ramp 80 provides tactile feedback to the user in both feel and sound. The user then knows that the cutting cannula 24 is armed. When the cutting cannula 24 is in the armed state as shown in Fig. 6, the cutting cannula 24 is retracted such that the spoon 23 is exposed as well as the tip of the stylet 20. The tip of the cutting cannula 24 resides at or near the rear of the spoon 27.

Referring to Fig. 7, the biopsy device 10 is shown in the armed position for the coring cannula 22. To move the biopsy device 10 to this position from the arming of the cutting cannula 24 of Fig. 6, the user continues pulling the plunger 34 rearwardly, which causes the followers 118 to contact the second deflectors 98, which deflects the plunger fingers 116 inwardly such that the strikes 120 extend into the catches or openings 158 of the coring cannula hub 150, thereby coupling the plunger fingers 116 to the coring cannula hub 150. The continued rearward movement of the plunger 34 deflects the deflectable stops 89 out of the way of the coring cannula hub 150, which then moves along with the coring cannula 22, rearwardly until the coring cannula hub 150 contacts the rearward stops 88, which provides tactile feedback to the user. Since the coring cannula hub 150 slides over the exterior of the body 172 of the cutting cannula hub 170, the coil spring 138 is not further compressed and its compression is independent of the movement of the coring cannula hub 150.

In the position of Fig. 7, where the coring cannula 22 is armed and the cutting cannula 24 is armed, the coring cannula 22 is withdrawn into the cutting cannula 24 to expose the tip of the stylet 20. As illustrated, the tip of the coring cannula 22 lies beyond the tip of the cutting cannula 24, but this need not be the case. As the stylet 20 now closes the coring cannula 22, the device is fully armed and ready for insertion into a tissue mass.

As an aside, it should be noted that the position of the stylet 20 can be altered by the optional specimen length selector 36. As shown in Fig. 7, the specimen length selector 36 is in the first detent 40. If the specimen length selector 36 were moved to the second detent 40, the tip of the stylet 20 would project further forward as shown in Fig. 7, which would reduce the length of the specimen. While only two detents 40 are illustrated, more are contemplated for a finer adjustment in the length of the specimen.

Referring to Fig. 8, after the biopsy device 10 is fully charged as shown in Fig. 7, it is ready for insertion into a tissue mass, where it can be guided to the desired location by suitable imaging device, such as ultrasound. Fig. 8 illustrates the coring cannula 22 being advanced forward to form a whole or partial specimen core, depending on the type of coring cannula. With the spoon-type coring cannula, a partial specimen core is formed. To move the biopsy device 10 into this position, the user pushes the plunger 34 forward to move forward the coring cannula hub 150 and along with it the coring cannula 22. The coring cannula hub 150 continues to move forward with the plunger 34 until the followers 118 reach the second relief channels 92, which provide for the deflection of the plunger fingers 116 and the disengagement of the strikes 120 from the openings 158 of the coring cannula hub 150, thereby disengaging the plunger 34 and the coring cannula hub 150.

In this position, the user will feel and hear this disengagement, which provides tactile feedback that the coring cannula 22 is now fully advanced. Any further advancement of the coring cannula hub 150 is prevented by the forward stops 86. However, in this position, the wedges 188 of the cutting cannula hub 170 reside within the openings 158 to provide a deflection surface for the plunger fingers 116 to more easily move forwardly beyond the coring cannula hub 150 upon subsequent movement of the plunger 34.

In the position of Fig. 8, the coring cannula 22 is now extended beyond the tip of the stylet 20. As shown, the spoon 27 is mostly extended beyond the end of the stylet 20. A portion of the stylet 20 is left within the spoon 27 to prevent any inadvertent tissue from prolapsing into the cutting cannula 24.

At this point, the user can immediately continue on with advancing the plunger 34 to complete the taking of the tissue specimen or the user can pause to readjust the position of the coring cannula 22 as they prefer.

Referring to Fig. 9, the biopsy device is shown at the completion of the taking of the specimen sample. To move into the position shown in Fig. 9 from that of Fig. 8, the user continues to advance the plunger 34, which causes the key tip 124 to pass between the interstitial rib 200 and the deflectable ramp 80 to thereby deflect the deflectable ramp 80 an amount sufficient to disengage the split deflector ramp 198 from the deflectable ramp 80. Once disengaged, the spring force from the coil spring 138 automatically advances the cutting cannula hub 170 until it contacts the nose. The advancement of the cutting cannula hub 170 advances the cutting cannula 24 over the spoon 27 of the coring cannula 22 thereby moving the cutting finger 25 into the window 26 to sever the tissue sample, which is contained within the coring cannula 22.

In terms of the biopsy device 10, the position of Fig. 9 is the same as the rest state of Fig. 5, with the exception that a tissue specimen now resides within the coring cannula 22. To remove the tissue specimen, the user moves the biopsy device 10 to the armed position for the cutting cannula 24, which exposes the spoon 27 thereby permitting the user to grasp the tissue specimen. If another sample is desired, the user just repeats the steps as described. If no further tissue specimens are desired, the user can place the biopsy device 10 back in the rest state by advancing the plunger 34.

Figs. 10-21 illustrate a second embodiment of a full core biopsy device that has many similarities to the first embodiment. Therefore, in many cases, like parts will be identified with like numbers increased by 200, with the understanding that the description of like parts in one of the embodiments can inform the understanding of the other embodiments. The basic structure and operation is similar for both the first and second embodiments. Differences will be easily seen or understood and in some cases expressly noted.

Referring to Fig. 10, there is illustrated a second embodiment of a full core biopsy device 210. The full core biopsy device 210 can include an actuator assembly 212 structurally and operably connected to a needle assembly 214. The needle assembly 214 can be utilized to penetrate a tissue mass for obtaining a core biopsy sample, alternately referred to as a biopsy specimen, from a target area in the tissue mass, such as a lesion, much like the first embodiment. The needle assembly includes a stylet 220, coring cannula 222, and cutting cannula 224. The coring cannula 222 can have a window 226 and the cutting cannula 224 can have a cutting finger 225 (Fig. 13) that passes through the window 226 to sever and retain a tissue sample in the coring cannula 222. The coring cannula 222 can further have a spoon cannula 227 on which the tissue sample may rest. One or both of the coring cannula 222 or the cutting cannula 224 can have a sharpened tip.

The actuator assembly 212 is a manually operated, single action hand-held device capable of controlling the acquisition and removal of a tissue specimen, which can be a core biopsy sample from the lesion site in the tissue mass. One difference between the second embodiment and the first embodiment is that the second embodiment includes a dwell between the completion of the advancement of the coring cannula 222, when forming the partial or full tissue core, and the firing of the cutting cannula 224 to automatically advance the cutting cannula 224 to sever the partial or full tissue core from the tissue mass. The dwell permits the user to confirm the position or reposition of the coring cannula 222 prior to manual actuation of the cutting cannula 224. The dwell is accomplished by permitting a nominal amount or more of free travel of the actuator assembly 212 from the point where the coring cannular 222 has reached its desired coring location and before the cutting cannula 224 is actuated or fired.

The actuator assembly 212 comprises a body 230, which can function as a grip or handle, from which a pair of finger grips 232 extend. A manual actuator, in the form of a plunger 234 extends from the body 230 opposite the needle assembly 214. While not shown, the actuator assembly 212 can, like the first embodiment, have an optional specimen length selector. The specimen length selector can be identical to the first embodiment. The plunger 234 can be manually advanced in a two-handed operation or a one-handed operation. In the two-handed operation, the user can grasp the body 230 with one hand and manually advance the plunger 234 with the other hand, or, alternatively, place their fingers of one hand in the finger grips 232 and use the other hand to move the plunger 234. In the single-handed operation, the user can place their fingers of one hand in the finger grips 232 and use the thumb of the same hand to move the plunger 234.

The biopsy device 210 has an operational axis X defined by the needle assembly 214 and extending through the actuator assembly 212. The axis X also defines an insertion axis of the needle assembly 214 into the tissue mass. The axis X need not be centered on the actuator assembly 212. As shown, the axis X is off center of the geometric center of the end of the actuator assembly 212.

Referring to Figs. 11A and 11B, the major elements of the actuator assembly 212 are better seen and understood. The body 230 is formed by a housing having an upper housing 250 and a lower housing 252. A series of spring clips in the form of a finger 254 and tab 256 are provided to secure together the upper housing 250 and the lower housing 252. The finger 254 has a slot 258 that receives the tab 256. The finger 254 is resilient, permitting it to deflect over the tab 256 to position the tab within the slot 258 when the upper and lower housings 250, 252 are snapped together, as seen in Fig. 10. When snapped together, the upper and lower housings 250, 252 define an interior 260.

Referring to Fig. 11B, the upper housing 250 has an end opening 251 at a rear end and a needle guide 272, at the front end or nose of the body 230. A pair of sidewalls 253 are located on opposite sides of upper housing 250. A follower channel 255 and follower deflector 257 are formed in the sidewalls 253 A hub stop 259 is located on and projects away from an interior surface of the upper housing 250. The hub stop 259 is located between the pair of sidewalls 253.

Referring to Fig. 11A, the lower housing 252 has an end plate 270, at a rear end. Arm channels 274 are located on opposite sides of the end plate 270. A stylet hub 330 and a spring tube seat 276 is provided on the end plate 270. A pair of sidewalls 284 are located on opposite sides of lower housing 252. First and second relief channels 290, 292 are formed in the sidewalls 284 of the lower housing 252 and are connected to the sidewalls 284 by first and second plunger deflectors 296, 298.

The plunger 234 comprises a pair of plunger arms 310 and a pair of keys 322. The plunger arms 310 are connected by a plunger hub 312 from which a nob 314 extends. The plunger arms 310 terminate in fingers 316 having an outwardly directed follower 318 and an inwardly directed coring hub strikes 320. Keys 322 extend from the plunger hub 312 and include releases 326 and catches 324. The keys 322 extend though arm channels 274. The releases 326 are illustrated as a ramp or deflector angled inward. However, the releases 326 may be a longer or shorter ramp of variable shape or curve to effect the timing of the release of the cutting cannula and level of resistance felt by the user.

A biasing device is provided in the handle and comprises a spring tube 334, which seats within the spring tube seat 276. A spring guide 336 is received within the spring tube 334, and a coil spring 338 is wound about the spring guide 336, which terminates in a head 337 against which the coil spring 338 bears. The spring guide 336 is biased by the coil spring 338 forward from a front face of the spring tube 334.

A coring cannula hub 350 has a body 351 defining a coring cannula passage 352 and having a coring cannula seat 354 having a through passage 356. A pair of resilient wings 355 extend away from the body 351 and have plunger catches 357 in the form of catch openings 358. A rear end of the coring cannula 222 is mounted to the coring cannula seat 354, such as by press-fitting the coring cannula 222 into the seat 354.

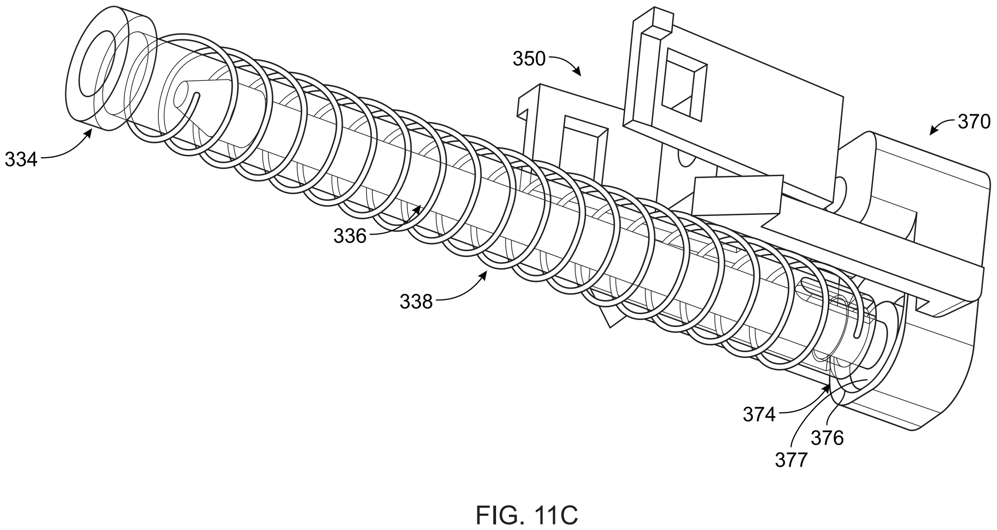

A cutting cannula hub 370 comprises a body 372 with a spring guide receiver 374 in the form of an internal channel 376, as seen in Fig. 11C, which receives the head 337 of the spring guide 336, and a cutting cannula seat 378 circumscribing a through passage 380. A pair of resilient catches 382 extend from the side of the body 372. The resilient catches 382 include resilient prongs 384 that terminate in tips 386. A set of wedges 388 extend from the body 372 at a location rearward of the resilient catches 382. The cutting cannula 224 can be mounted to the cutting cannula seat 378, such as, by press-fitting a rear end of the cutting cannula 224 into the cutting cannula seat 378.

Referring to Fig. 12, the actuator assembly 212 is shown with the upper housing 250 removed to better see the locations of the plunger 234, stylet hub 330, coring cannula hub 350, and cutting cannula hub 370 when the actuator assembly 212 is in condition where the spoon of the coring cannula 222 is exposed to illustrate the parts. When the biopsy device 210 is assembled, a rear end of the stylet 220 is press-fit within the stylet hub 330 and passes through the through passages 356 and 380 of the coring cannula hub 350 and the cutting cannula hub 370. The coring cannula 222 is press-fit within the coring cannula seat 354 and passes through the through passage 380 of the cutting cannula hub 370. The cutting cannula 224 is press-fit within the cutting cannula seat 378.

In this configuration, the stylet 220 is located within the lumen of the coring cannula 222, which is located within the lumen of the cutting cannula 224. Thus, the relative sliding movement of the coring cannula hub 350 and the cutting cannula hub 370 results in the relative movement of the coring cannula 222 and cutting cannula 224 to the stylet 220 and to each other. The spring tube 334 resides on a lower portion of the end plate 270 below the stylet 220 and projects forward toward the nose of the body 230. The coil spring 338 may be disposed over or around the spring tube 334 and applies a biasing force against spring guide 336 which is inserted into the internal channel 376 of the cutting cannula hub 370.

The coring cannula hub 350 is positioned within the lower housing 252 such that it resides between the stylet hub 330 and the cutting cannula hub 370. In this position, the through passage 356 is in alignment with the stylet seat 340 and the stylet 220 can pass through the through passage 356 and into the interior of the coring cannula 222.

The cutting cannula hub 370 is positioned within the lower housing 252 such that it resides between first and second relief channels 290, 292. The body 372 of the cutting cannula hub 370 is aligned with and free to move over the coring cannula passage 352. The cutting cannula through passage 380 is aligned with the coring cannula through passage 356 thereby permitting the insertion of both the coring cannula 222 and the stylet 220 residing within the cutting cannula 224.

The operation of the biopsy device 210 will be described with reference to Figs. 13-19. For Figs. 13-19, only the part numbers relevant to the corresponding description are illustrated in the drawing to avoid overcrowding the drawing with part numbers to improve the view of the part. It should be noted that for Figs. 13-19, the enlarged needle end is rotated 90 degrees relative to the housing to better see the interaction between the excising finger and the window.

In Fig. 13, the biopsy device 210 is shown in a rest state, where the plunger 234 is fully inserted into the handle such that the followers 318 are received within the follower channel 255 and have not engaged the coring cannula hub 350 catch openings 358. In this position movement of the cutting cannula hub 370 is independent of the coring cannula hub 350 because hub strikes 320 of the plunger 234 are not engaged with the prongs 384 while the catches 324 of the plunger 234 abut the cutting cannula hub 370. Thus, any withdrawal of the plunger 234 from this position will only initially move the cutting cannula hub 370 as the catches 324 bear against and move the cutting cannula hub 370. When in this position, the plunger fingers 316 are substantially undeflected, the coil spring 338 is mostly uncompressed, yet still applying some biasing force against the cutting cannula hub 370, the prongs 384 are mostly un-sprung because the tips 386 are located within the first relief channels 290, and the coring cannula hub 350 lies between the hub stop 259 and the stylet hub 330.

In this position, the coring cannula 222 and cutting cannula 224 are fully forward, with the cutting finger 225 of the cutting cannula 224 extending through the window 226 of the coring cannula 222. The tip of the stylet 220 is not visible and is within the coring cannula 222, spaced axially from the spoon cannula 227.

In Fig. 14, the biopsy device 210 is shown starting an arming cycle (also referred to as cocking, charging, etc.), where the plunger 234 is engaged with both the cutting cannula hub 370 and the coring cannula hub 350, such that further movement of the plunger 234 will move both hubs simultaneously. To get to this position from Fig. 13, the plunger 234 is partially withdrawn to the point where the hub strikes 320 engage the openings 358 of the wings 355. In moving to this position, the cutting cannula hub 370 is moved rearwardly when the catches 324 contact the cutting cannula hub 370 which simultaneously causes the tips 386 of the prongs 384 to leave the first relief channels 290 and begin to ride along the deflector 298, which starts to deflect the prongs 384 inwardly.

For the hub strikes 320 to engage the openings 358 of the wings, the followers 318 deflect inwardly and ride over the deflectors 257 in the upper cover 250, and are then received within the follower channel 255, which will cause tactile feedback and an audible click for the user, which lets them know the position of the plunger 234. In this position, the hub strikes 320 are adjacent to the coring hub 350 catch openings 358. It should be noted that the deflectors 257 extend downward from the cover and into the path of the followers 318.

In this position the plunger fingers 316 are substantially undeflected, the coil spring 338 is abutting and biasing the cutting cannula hub 370 forward into abutment with the catches 324, and the coring cannula hub 150 lies between the hub stop 259 and the stylet hub 330.

Also in this position, the cutting finger 225 is withdrawn from the fully forward position and from the window 226 of the coring cannula 222, and the tip of the stylet 220 is not visible and is within the coring cannula 222, spaced axially from the spoon cannula 227.

Fig. 15 illustrates the continued arming where, since the hub strikes 320 have now engaged the openings 358 in the coring cannula hub 350, further withdrawal of the plunger 234 simultaneously moves both of the cutting cannula hub 370 and the coring cannula hub 350 to further draw both of the cutting cannula 224 and coring cannula 222 rearwardly relative to the stylet 220. This further withdrawal of the plunger 234 to the position of Fig. 15 results in the receipt of the followers 318 within the follower channel 255 of the upper housing 250.

In this position the plunger fingers 316 start to deflect inward into openings 358 of coring cannula hub 350 and engage openings 358 and thereby moving the coring cannula hub 350 in concert with the plunger 234. The coil spring 338 is abutting and biasing the cutting cannula hub 370 forward into abutment with the catches 324, the prongs 384 are deflected further inward as they follow the deflector 298.

Also, in this position, the coring cannula 222 is still fully forward, in the same position as Fig. 14, and has not yet started to move rearwardly. However, any additional withdrawal of the plunger 234 will start to move the coring cannula hub 350 as well as the cutting cannula hub 370. In this position, the cutting cannula 224 and the cutting finger 225 are further withdrawn relative to the window 225 of the coring cannula. The tip of the stylet 220 is not visible and is within the coring cannula 222, spaced axially from the spoon cannula 227.

Fig. 16 illustrates the biopsy device 210 in the fully armed state where the plunger 234 is fully withdrawn and simultaneously moving with it both of the cutting cannula 224 and the coring cannula 222 until the tips 386 of the prongs 384 reach the second relief channels 292, where the resiliency of the sprung prongs 384 forces the tips 386 into the second relief channels 292 and locks the cutting hub 370 in the fully armed position. In this fully armed position, the forward movement of the plunger 234 will only move the coring cannula hub 350, since the receipt of the tips 386 of the prongs 384 in the second relief channels 292 have temporarily locked the position of the cutting cannula hub 370, causing the hub strikes 320, which are already received within the openings 358 of the wings 355, to drive the coring cannula 222 forward in concert with the plungers 234 movement.

It is contemplated that the coring cannula hub 350 abuts the end plate 270 at the same time the tips of the wings 355 are received in the second relief channels 292, which provides tactile feedback to the user in both the contact of the hub 350 and the end plates 270 along with the feel and sound of the tips 386 snapping into the second relief channels 292. However, it is not necessary that these two sources of tactile feedback happen at the same time.

In the position of Fig. 16, the coring cannula 222 and cutting cannula 224 are withdrawn together yet relative to the stylet 220 to expose the tip of the stylet 220. As illustrated, the tip of the coring cannula 222 lies beyond the tip of the cutting cannula 224, but this need not be the case. As the stylet 220 now closes the coring cannula 222, the device is fully armed and ready for insertion into a tissue mass.

Fig. 17 is a longitudinal sectional view of the second embodiment biopsy device 210 when the plunger 234 is advanced forward to the beginning of a dwell state, where the plunger 234 is advanced to a point where the coring cannula 222 advances relative to both the stylet 220 and the cutting cannula 224 to extend the coring cannula 222 and expose the spoon 227 beyond the end of the cutting cannula 224 and stylet 220. However, the coring cannula 222 is not advanced so far as to trigger the actuation of the cutting cannula 224.

In movement to this position from that of Fig. 16, the plunger 234 is advanced moving with it the coring cannula 222, but not the cutting cannula 224, which is still latched. The plunger 234 is advanced to the point where the hub strikes 320 are abutting the tips 386 of the wings 355. The user will feel this contact and know they have reached the dwell. In this position, the user can readjust the position of the coring cannula, if desired. The user could also partially or fully withdraw the plunger 234 to partially or fully restart the process if the spoon 227 of the coring cannula 222 is not in the desired position.

At this point the hub strikes 320 are released from the openings 358 in the wings 355 and the coring cannula hub 350 is abutting the hub stop 259, any further forward movement of the plunger 234 will not advance the coring cannula hub 350 further. In this position, the coring cannula 222 is advanced relative to the cutting cannula 224 to expose the spoon 227, which, when in the tissue mass, will insert the spoon 227 into the tissue.

Fig. 18 illustrates the point of actuation of the cutting cannula 224. From the position of Fig. 17, the plunger 234 is further advanced to drive release 326 into wedges 388. The interaction of the release 326 and wedges 388 surfaces as the plunger 234 is advanced causes the prongs 384 to deflect inwardly until the tips 386 of the prongs 384 are free of the second relief channels 292. Fig. 18 illustrates the point where the tips 386 are just free of the second relief channels 292. At this point, the force of the compressed spring 338 will drive the cutting cannula hub 370 forward and along with it the cutting cannula 224 to sever the tissue mass.

After the biopsy device 210 is fully charged as shown in Fig. 16, it is ready for insertion into a tissue mass, where it can be guided to the desired location by a suitable imaging device, such as an ultrasound. Fig. 18 illustrates the coring cannula 222 being advanced forward to form a whole or partial specimen core, depending on the type of coring cannula. With the spoon-type 227 coring cannula 222, a partial specimen core is formed. To move the biopsy device 210 into this position, the user pushes the plunger 234 forward to move forward the coring cannula hub 350 and along with it the coring cannula 222. The coring cannula hub 350 continues to move forward with the plunger 234 until the followers 318 reach the second relief channels 292, which provide for the deflection of the plunger fingers 316 and the disengagement of the hub strikes 320 from the openings 358 of the coring cannula hub 350, thereby disengaging the plunger 234 and the coring cannula hub 350.

In this position, the user will feel and hear this disengagement, which provides tactile feedback that the coring cannula 222 is now fully advanced. However, in this position, the wedges 388 of the cutting cannula hub 370 reside within the second relief channels 292 to provide a deflection surface for the release 326 of the plunger 234 to more easily move forward pushing the wedges 388 out of the second relief channel 292 upon subsequent movement of the plunger 234. The user will feel an increase in resistance to forward movement as pressure from the release 326 drives the wedges 388 inwardly towards each other.

In the position of Fig. 18, the coring cannula 222 is now extended beyond the tip of the stylet 220. As shown, the spoon 277 of the coring cannula 222 is mostly extended beyond the end of the stylet 220. A portion of the stylet 220 is left within the spoon 227 of the coring cannula 222 to prevent any inadvertent tissue from prolapsing into the cutting cannula 224.

At this point, the user can immediately continue on with advancing the plunger 234 to complete the taking of the tissue specimen or the user can pause to readjust the position of the coring cannula 222 as they prefer.

Referring to Fig. 19, the biopsy device is shown at the completion of the taking of the specimen sample, that is, after the force of the compression spring 338 biases the cutting hub 370 forward. Forward movement of the plunger 234 moves the release 326 to completely removed the wedges 388 of prongs 384 of the cutting cannula hub 370, out of the second relief channel 292. The spring force from the coil spring 338 automatically advances the cutting cannula hub 370 until it contacts the nose. The advancement of the cutting cannula hub 370 advances the cutting cannula 224 over the spoon cannula 227 of the coring cannula 222 thereby moving the cutting finger 225 into the window 226 to sever the tissue sample, which is contained within the coring cannula 222.

In terms of the biopsy device 210, the position of Fig. 19 is the same as the rest state of Fig. 13, with the exception that a tissue specimen now resides within the coring cannula 222. To remove the tissue specimen, the user moves the biopsy device 210 to the armed position resulting in the stylet pushing the specimen out of the coring cannula 222 and the cutting cannula 224.. If another sample is desired, the user just repeats the steps as described. If no further tissue specimens are desired, the user can place the biopsy device 210 back in the rest state by advancing the plunger 234.

While the first and second embodiments are described with a cutting finger 25, 225 that passes through a window 26, 226 in the coring cannula 22, 222, the cutting finger 25, 225 need not pass through a window 26, 226 but can pass over the end of the coring cannula 22, 222, using the inherent resiliency of the cutting finger 25, 225, instead of the physical deflection through the window 26, 226, to sever the tissue sample. Similarly, while the coring cannula 222 is illustrated with a spoon cannula 227, it need not have a spoon. Fig. 20 illustrates one such alternative where the coring cannula 322 can be a complete cylinder with or without a sharpened tip and the cutting finger 325 can pass over the tip. In some cases, the tip can be castellated, as seen in Fig. 20, defining a series of merlons separated by embrasures, with the merlons being in the shape of sharpened points, and the cutting finger passing in front of or through one or more of the embrasures. Fig. 21 illustrates another alternative needle assembly where the coring cannula 422 does not have a spoon, but does have a window 426 through which the finger 425 passes.

This written description uses examples to describe aspects of the disclosure described herein, including the best mode, and also to enable any person skilled in the art to practice aspects of the disclosure, including making and using any devices or systems and performing any incorporated methods. The patentable scope of aspects of the disclosure is defined by the claims, and can include other examples that occur to those skilled in the art. Such other examples are intended to be within the scope of the claims if they have structural elements that do not differ from the literal language of the claims, or if they include equivalent structural elements with insubstantial differences from the literal languages of the claims.

Further aspects are provided by the subject matter of the following clauses:
1. A biopsy device for taking a soft tissue specimen core sample from a soft tissue mass within a body, the biopsy device comprising: a needle assembly comprising a stylet, a coring cannula, and a cutting cannula, with the stylet received within the coring cannula, and the coring cannula received within the cutting cannula, wherein the coring cannula can be advanced to a coring position to form a specimen core and the cutting cannula can be advanced to a severing position to sever the specimen core from the tissue mass; and a single action actuator assembly to move the needle assembly between the coring position and severing position and comprising a manually operable actuator to advance the coring cannula to the coring position and an automatic firing of the cutting cannula to advance the cutting cannula to the severing position.
2. The biopsy device of any preceding clause, wherein the manual advancement of the coring cannula can be paused prior to the automatic firing of the cutting cannula.
3. The biopsy device of any preceding clause, wherein the manual advancement of the coring cannula moves the coring cannula beyond a tip of the stylet.
4. The biopsy device of any preceding clause, wherein the stylet is stationary during the manual advancement of the coring cannula.
5. The biopsy device of any preceding clause, wherein the biopsy devices comprises a housing supporting the needle assembly and the manually operable actuator comprises a plunger moveable relative to the housing and operably coupled to the coring cannula wherein movement of the plunger advances the coring cannula.
6. The biopsy device of any preceding clause, wherein the movement of the plunger initiates the automatic firing of the cutting cannula.
7. The biopsy device of any preceding clause, wherein the movement of the plunger initiates the automatic firing of the cutting cannula upon the coring cannula reaching the coring position.
8. The biopsy device of any preceding clause, wherein the movement of the plunger initiates the automatic firing of the cutting cannula upon movement of the plunger after the coring cannula reaching the coring position.
9. The biopsy device of any preceding clause, wherein the single action actuator comprises a dwell where the plunger is advanced a predetermined distance after the coring cannula reaches the coring position to initiate the automatic firing.
10. The biopsy device of any preceding clause, wherein the needle assembly is coaxial about a first axis and the plunger is moved about a second axis, which is parallel to the first axis.
11. The biopsy device of any preceding clause, wherein the second axis is coaxial with the first axis.
12. The biopsy device of any preceding clause, wherein the specimen core is either a whole specimen core or a partial specimen core.
13. The biopsy device of any preceding clause, wherein the coring cannula is a spoon cannula.
14. A biopsy device for taking a soft tissue specimen core from a soft tissue mass within a body, the biopsy device comprising:
   a needle assembly comprising
   a stylet,
   a coring cannula, and
   a cutting cannula, with the stylet received within the coring cannula, and the coring cannula received within the cutting cannula; and
   a single action actuator assembly comprising:
      a housing carrying the needle assembly,
      an automatic actuator operable, after being triggered, to automatically move the needle assembly to a severing condition to sever a specimen core carried by the coring cannula from the tissue mass, and
      a manual actuator operable to move the needle assembly from an armed condition to a coring condition where a specimen core is formed in the coring cannula and a triggered condition where the automatic actuator is triggered.
15. The biopsy device of clause 14, wherein the manual actuator comprises a plunger slidably mounted to the housing for reciprocal movement between the triggered condition and the armed condition.
16. The biopsy device of clause 15, wherein the plunger moves the needle assembly to the coring condition as the plunger moves between the armed condition and the triggered condition.
17. The biopsy device of any of clauses 14-16, wherein the coring condition coincides with the triggered condition.
18. The biopsy device of any of clauses 14-17, wherein the single action actuator comprises a detent at the coring condition.
19. The biopsy device of clause 15 or 16, or any of clauses 17-18 when dependent on clause 15, wherein the needle assembly defines a first axis and the plunger slides along a second axis that is parallel to the first axis.
20. The biopsy device of clause 19, wherein the first axis and second axis are coaxial.

While the invention has been specifically described in connection with certain specific embodiments thereof, it is to be understood that this is by way of illustration and not of limitation. Reasonable variation and modification are possible with the scope of the foregoing disclosure and drawings without departing from the spirit of the invention which, is defined in the appended claims. Hence, specific dimensions and other physical characteristics relating to the embodiments disclosed herein are not to be considered as limiting, unless the claims expressly state otherwise.

## Claims

1. A biopsy device for taking a soft tissue specimen core sample from a soft tissue mass within a body, the biopsy device comprising:
a needle assembly comprising a stylet, a coring cannula, and a cutting cannula, with the stylet received within the coring cannula, and the coring cannula received within the cutting cannula, wherein the coring cannula can be advanced to a coring position to form a specimen core and the cutting cannula can be advanced to a severing position to sever the specimen core from the tissue mass; and
a single action actuator assembly to move the needle assembly between the coring position and severing position and comprising a manually operable actuator to advance the coring cannula to the coring position and an automatic firing of the cutting cannula to advance the cutting cannula to the severing position.

2. The biopsy device of claim 1, wherein the manual advancement of the coring cannula can be paused prior to the automatic firing of the cutting cannula.

3. The biopsy device of any preceding claim, wherein the manual advancement of the coring cannula moves the coring cannula beyond a tip of the stylet.

4. The biopsy device of claim 3, wherein the stylet is stationary during the manual advancement of the coring cannula.

5. The biopsy device of any preceding claim, wherein the biopsy device comprises a housing supporting the needle assembly and the manually operable actuator comprises a plunger moveable relative to the housing and operably coupled to the coring cannula wherein movement of the plunger advances the coring cannula.

6. The biopsy device of claim 5, wherein the movement of the plunger initiates the automatic firing of the cutting cannula.

7. The biopsy device of claim 6, wherein the movement of the plunger initiates the automatic firing of the cutting cannula upon the coring cannula reaching the coring position.

8. The biopsy device of claim 6, wherein the movement of the plunger initiates the automatic firing of the cutting cannula upon movement of the plunger after the coring cannula reaches the coring position.

9. The biopsy device of claim 8, wherein the single action actuator comprises a dwell where the plunger is advanced a predetermined distance after the coring cannula reaches the coring position to initiate the automatic firing.

10. The biopsy device of any of claims 5-9, wherein the needle assembly is coaxial about a first axis and the plunger is moved about a second axis, which is parallel to the first axis.

11. The biopsy device of claim 10, wherein the second axis is coaxial with the first axis.

12. The biopsy device of any preceding claim, wherein the specimen core is either a whole specimen core or a partial specimen core.

13. The biopsy device of any preceding claim, wherein the coring cannula is a spoon cannula.
